# EUROPÄISCHE PATENTSCHRIFT

(11) **EP 0 888 321 B1**
(45) Veröffentlichungstag und Bekanntmachung des Hinweises auf die Patenterteilung: **10.11.1999**
(21) Anmeldenummer: 97901074.1
(22) Anmeldetag: 22.01.1997
(51) Int. Cl.: C07D 301/00, C07D 303/04

(54) **VERFAHREN ZUR SELEKTIVEN HYDRIERUNG VON VINYLOXIRAN ZU 1,2-BUTYLENOXID AN HETEROGENKATALYSATOREN**
PROCESS FOR THE SELECTIVE HYDRATION OF VINYLOXIRANE TO 1,2-BUTYLENE OXIDE ON HETEROGENEOUS CATALYSTS
PROCEDE D'HYDRATATION SELECTIVE DE VINYLOXYRANE SUR DES CATALYSEURS HETEROGENES POUR PRODUIRE DU 1,2-BUTYLENE OXYDE

(30) Priorität: 26.01.1996 DE 19602710
(43) Veröffentlichungstag der Anmeldung: 07.01.1999
(73) Patentinhaber: BASF AKTIENGESELLSCHAFT, 67056 Ludwigshafen (DE)
(72) Erfinder: SIGWART, Christoph, D-69198 Schriesheim (DE); HEINEKE, Daniel, D-67065 Ludwigshafen (DE); FLICK, Klemens, D-76863 Herxheim (DE)
(86) Internationale Anmeldenummer: EP9700281
(87) Internationale Veröffentlichungsnummer: WO9727182

(56) Entgegenhaltungen:
- EP-A- 0 653 243
- EP-A- 0 724 907
- DE-A- 4 407 486
- DE-A- 4 422 046
- US-A- 5 077 418
- ANGEWANDTE CHEMIE, INTERNATIONAL EDITION, Bd. 30, Nr. 10, Oktober 1991, WEINHEIM, DE, Seiten 1312-4, XP002028899 H. BÖNNEMANN ET AL.: "Formation of colloidal transition metals in organic phases and their application in catalysis" in der Anmeldung erwähnt

## Beschreibung

Die vorliegende Erfindung betrifft ein verbessertes Verfahren zur Herstellung von 1,2-Butylenoxid durch katalytische Hydrierung von Vinyloxiran an Heterogenkatalysatoren.

Die heterogen-katalytische Hydrierung von Vinyloxiran ist bekannt.

Nach der US-A 2,561,984 entsteht bei der Hydrierung von Vinyloxiran in Ethanol an einem Palladium-Aktivkohle-Katalysator bei 25°C/2 bar nach einer Reaktionszeit von 3 h als Hauptprodukt n-Butyraldehyd. Mit Raney-Nickel als Katalysator wird dagegen bei 25°C und 2 bar nach einer Reaktionszeit von 1,5 h hauptsächlich n-Butanol gebildet. Über die Bildung von 1,2-Butylenoxid wird nicht berichtet.

In einer Arbeit von Aizikovich et al. (J. Gen. Chem. USSR, 28 (1958) 3076) wird die katalytische Hydrierung von Vinyloxiran in Methanol bzw. Ethanol an Platin-, Palladium- und Raney-Nickel-Katalysatoren beschrieben. Mit einem Palladium-Trägerkatalysator (1,8 Gew.-% Palladium auf Calciumcarbonat) wird bei 15°C/1 bar hauptsächlich n-Butanol gebildet. Als wichtigste Zwischenverbindung der Hydrierung wird in dieser Schrift Crotylalkohol angesehen, obwohl auch die Bildung von n-Butyraldehyd beobachtet wird. Zur Bildung von 1,2-Butylenoxid gibt es auch in dieser Arbeit keine Hinweise.

In den Schriften US-A 5,077,418 und US-A 5,117,013 wird berichtet, daß bei der Hydrierung von Vinyloxiran-Lösungen an Palladium-haltigen Katalysatoren als Hauptprodukt n-Butyraldehyd gebildet wird. So erhält man bei der Hydrierung von Vinyloxiran mit Tetrahydrofuran als Lösungsmittel an einem Palladium-Aktivkohle-Trägerkatalysator (5 Gew.-% Palladium auf Aktivkohle) bei einer Temperatur von 50 bis 55°C und einem Druck von 3,5 bar nach einer Reaktionszeit von 3 h einen Hydrieraustrag, der 55 % n-Butyraldehyd, nur 27 % 1,2-Butylenoxid und 9 % n-Butanol enthält. Wird die Hydrierung an Palladium-haltigen Aluminiumoxid-Trägerkatalysatoren (5 % Pd/Al₂O₃) durchgeführt, so werden bei einer Temperatur von 25 bis 55°C und einem Druck von 3,5 bar nach einer Reaktionszeit von 6 h bzw. bei einer Temperatur von 100°C und einem Druck von 20,7 bar nach einer Reaktionszeit von 4 h nur Spuren von 1,2-Butylenoxid gebildet. Bei quantitativem Umsatz entsteht als Hauptprodukt n-Butyraldehyd mit einer Selektivität von 87 % bzw. 78 %. Beide US-Anmeldungen beschreiben außerdem die Hydrierung von Vinyloxiran an Raney-Nickel, wobei als Hauptprodukt n-Butanol gebildet wird. Die 1,2-Butylenoxid-Ausbeute ist mit 41 % relativ niedrig. Bei der Hydrierung von Vinyloxiran an einem Platin-haltigen Trägerkatalysator (1 Gew.-% Pt/Al₂O₃) bei 100°C und 20,7 bar Wasserstoffdruck werden nach einer Reaktionszeit von 4,6 h bei vollständigem Umsatz nur 40 % 1,2- Butylenoxid neben 23 % n-Butanol, 24 % Butenolen, 5 % Crotonaldehyd und 3 % n-Butyraldehyd gefunden. Andere Platin-haltige Katalysatoren liefern noch geringere 1,2-Butylenoxid-Ausbeuten.

Die US-A 5,077,418 und US-A 5,117,013 lehren weiterhin, daß hohe 1,2- Butylenoxid-Ausbeuten nur mit Rhodium-haltigen Katalysatoren erhalten werden. Mit verschiedenen Rhodium-haltigen Trägerkatalysatoren (5 Gew.-% Rhodium auf Aktivkohle, 5 Gew.-% Rhodium auf Aluminiumoxid), die allerdings einen hohen Gehalt an dem teuren Edelmetall Rhodium haben, bzw. mit Rhodiumoxidhydrat (Rh₂O₃* x H₂O) werden bei der Hydrierung von Vinyloxiran-Lösungen 1,2-Butylenoxid-Gehalte von 60 bis 93 % erhalten. Nachteilig an diesem Verfahren ist die geringe Raum-Zeit-Ausbeute, bezogen auf die eingesetzte Rhodiummenge. So beträgt die Raum-Zeit-Ausbeute in Beispiel 2 von US-A 5,117,013 nur 110 kg 1,2-Butylenoxid/kg Rh * h.

In Neftekhimiya 33 (1993) 131 wird die Hydrierung von Vinyloxiran an Nickel-, Palladium- und Kupfer-haltigen Katalysatoren beschrieben. Mit Raney-Nickel bzw. Nickel auf Kieselgur als Katalysator verläuft die Hydrierung hauptsächlich unter Spaltung des Epoxid-Rings, die zur überwiegenden Bildung von 1-Butenolen und n-Butanol führt. Die Ausbeuten an Butylenoxid sind gering. Beispielsweise wird bei einem Hydrierversuch in Methanol bei 20°C/60 bar Wasserstoffdruck an einem mit Isopropanol, Nicotinsäure, Pyridin und Morpholin vorbehandelten Raney-Nickel-Katalysator bei 89 % Umsatz eine Butylenoxid-Selektivität von nur 37 % erreicht. Mit Palladium-haltigen Katalysatoren werden zwar im Vergleich zu den Versuchen mit Nickel-haltigen Katalysatoren höhere Butylenoxid-Selektivitäten erzielt. Beispielsweise wird mit einem Palladium/Aktivkohle-Katalysator ohne Einsatz eines Lösungsmittels bei 15°C/60 bar Wasserstoffdruck nach 13 min Reaktionszeit bei 61 %igem Umsatz 81 % Butylenoxid, bezogen auf umgesetztes Vinyloxiran, erhalten. Da Vinyloxiran und 1,2-Butylenoxid destillativ praktisch nicht voneinander getrennt werden können, kommt diesem Verfahren jedoch keine technische Bedeutung zu. Mit Kupfer-haltigen Katalysatoren wird eine niedrigere Hydrieraktivität und Verharzung des Hydrieraustrages beobachtet, die dieses Verfahren technisch impraktikabel macht.

Die deutsche Patentanmeldung P 44 22 046.4 betrifft Palladiumhaltige, durch Tränkung von bestimmten Trägermaterialien mittels Metallsalzen hergestellte Katalysatoren (Pd/BaSO₄, Pd/ZrO₂, Pd/TiO₂, Pd/Re/Träger) zur Verwendung in der selektiven Hydrierung von Vinyloxiran zu 1,2-Butylenoxid. Trotz der in dieser Schrift beschriebenen hohen Butylenoxid-Selektivitäten entstehen als Nebenprodukt relativ große Mengen an Butyraldehyd (4 bis 20 %).

Die DE-A 44 07 486 lehrt die Hydrierung von Vinyloxiran an Katalysatoren, die durch Aufdampfen der katalytisch aktiven Elemente auf einen Träger aus Metallfolie bzw. Metalldrahtgeweben erhalten werden. Diese Katalysatoren ermöglichen eine hochselektive Umsetzung zum gewünschten Verfahrensprodukt (1,2-Butylenoxid-Ausbeute: 75 bis 90 %), jedoch sind die eingesetzten Metallträger relativ teuer.

Die deutsche Patentanmeldung 19 532 645.8 betrifft die katalytische Hydrierung von Vinyloxiran an Heterogenkatalysatoren, die durch Gasphasenabscheidung von Elementen der Gruppen 7 bis 11 des Periodischen Systems der Elemente auf einem inerten, nichtmetallischen Träger hergestellt worden sind.

Der vorliegenden Erfindung lag somit die Aufgabe zugrunde, ein wirtschaftlicheres Verfahren zur Herstellung von 1,2-Butylenoxid aus Vinyloxiran zu finden, bei dem 1,2-Butylenoxid in hoher Ausbeute und Selektivität gebildet wird und bei dem die eingesetzten Hydrierkatalysatoren kostengünstig herstellbar sind, wobei insbesondere billige Trägermaterialien, einfache Aufbringung der Aktivkomponente und geringe Aktivkomponentenmengen gesucht waren.

Dementsprechend wurde ein Verfahren zur Herstellung von 1,2-Butylenoxid durch katalytische Hydrierung von Vinyloxiran an einem Heterogenkatalysator gefunden, das dadurch gekennzeichnet ist, daß man einen Katalysator verwendet, der durch Aufbringen mindestens eines Elements der Gruppe Palladium, Platin, Rhodium, Rhenium oder Ruthenium in Form eines Sols auf einen inerten Träger hergestellt wird.

Das erfindungsgemäße Verfahren ermöglicht es, die Doppelbindung des Vinyloxirans (I) gemäß Gleichung (1) selektiv zu hydrieren, 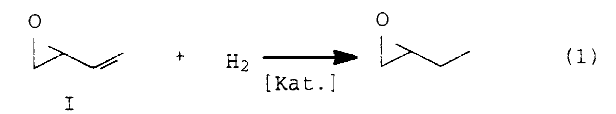 ohne daß der empfindliche Epoxidring bei der Hydrierung in nennenswertem Umfang hydrogenolytisch gespalten wird und ohne daß es in nennenswertem Umfang zu anderen Nebenreaktionen, z.B. Isomerisierung des Vinyloxirans, beispielsweise zu Crotonaldehyd, das in Folge zu Crotylalkohol und Butanol hydriert wird, kommt. Bei Verwendung von Rhodium-haltigen Katalysatoren können gegenüber den in US-A 5,077,418 und US-A 5,117,013 beschriebenen Rhodium-Katalysatoren höhere Produktivitäten, bezogen auf eingesetztes Edelmetall, erzielt werden.

Die erfindungsgemäß verwendeten Katalysatoren werden hergestellt, indem man Elemente der Gruppe Re, Ru, Rh, Pd und Pt als Sol auf einem inerten Täger aufbringt. Die erfindungsgemäßen Katalysatoren enthalten eines oder mehrere der genannten Metalle.

Die erfindungsgemäßen Katalysatoren haben bevorzugt einen Metallgehalt von 0,001 bis 2 Gew.-%, vorteilhaft beträgt der Metallgehalt 0,005 bis 0,5 Gew.-%, insbesondere 0,005 bis 0,1 Gew.-%, bezogen auf den Katalysator. Die Herstellung des Katalysators erfolgt durch Besprühen eines heißen Trägers oder durch Tränkung des Trägers mit einem zuvor hergestellten Sol. Das Metallsol ist ein kolloidaler Stoff und kann nach bekannten Methoden, z.B. ausgehend von Metallsalzen, in denen das Metall in einer Oxidationsstufe größer Null vorliegt, hergestellt werden. Es können z.B. wäßrige Lösungen der Chloride, Acetate oder Nitrate des Metalls verwendet werden. Es sind aber auch andere Metallsalze verwendbar; eine Beschränkung hinsichtlich des Anions besteht nicht. Als Reduktionsmittel lassen sich organische Verbindungen wie Ethanol, Methanol, Carbonsäuren und deren Alkalisalze sowie anorganische Verbindungen wie N₂H₄ oder NaBH₄ verwenden. Bevorzugt sind Hydrazin N₂H₄ und Ethanol. Die Teilchengröße der Metallpartikel im Sol hängt hierbei von der Stärke des eingesetzten Reduktionsmittels und vom verwendeten Metallsalz ab. Die Sole lassen sich durch Zugabe von organischen Polymeren wie Polyaminen, Polyvinylpyrrolidon oder Polyacrylaten stabilisieren, wobei Polyvinylpyrrolidon PVP bevorzugt ist. Die Solherstellung kann aber auch nach anderen in der Literatur beschriebenen Vorgehensweisen durchgeführt werden. Bönnemann et al. (Angew. Chemie, 103 (1991) 1344) beschreiben beispielsweise die Herstellung von stabilen Metallsolen durch Reduktion von Metallsalzen mit (C₈H₁₇)₄N[BEtH₃].

Als inerte Trägersubstanzen für die im erfindungsgemäßen Verfahren anwendbaren Katalysatoren kommen beispielsweise Formkörper aus Glas, Quarzglas, Keramik, Titandioxid, Zirkondioxid, Aluminiumoxid, Alumosilikate, Borate, Steatit, Magnesiumsilikat, Siliciumdioxid, Silikate, Kohlenstoff, z.B. Graphit oder Mischungen der genannten Materialien in Frage. Bevorzugt ist Aluminiumoxid. Der Träger kann porös oder nicht porös sein. Als Formkörper kommen Stränge, Tabletten, Wagenräder, Sterne, Monolithe, Kugeln, Splitt, Ringe oder Extrudate in Betracht. Besonders bevorzugt sind Kugeln, Tabletten und Stränge.

In der Regel haben die inerten Träger BET-Oberflächen von 0 bis 1000 m²/g bevorzugt von 0,1 bis 400 m²/g.

Die Aufbringung der Sole auf den Träger kann nach verschiedenen Techniken erfolgen, die die Verteilung der Aktivkomponente beeinflussen. Zur Erzeugung dünner Schalen der Aktivkomponente für den gesamten Strangquerschnitt wird das Sol auf einen indirekt beheizten Träger aufgesprüht. Man geht dabei so vor, daß der Träger in einem drehbaren, beheizbaren Pelletierteller vorgelegt und durch ein Heißluftgebläse auf Temperaturen zwischen 80 und 200°C aufgeheizt wird. Während der Teller gedreht wird, sprüht man das Sol auf den Träger auf. Durch das Drehen des Tellers wird die Durchmischung der Trägerteilchen, z.B. Stränge oder Splitt, gewährleistet. Beim Kontakt mit dem heißen Träger verdampft die Flüssigkeit im Sol und die Aktivkomponente bleibt auf dem Träger zurück. Durch diese Aufbringtechnik entstehen Katalysatoren, in denen die Aktivkomponente in dünnen Schichten, die im allgemeinen kleiner als 5 µm sind, auf dem Träger aufgebracht ist. Die Teilchengröße der Edelmetallagglomerate liegt im allgemeinen in derselben Größenordnung wie im Sol. Der Katalysator wird dann bei einer Temperatur, die 150°C nicht überschreitet, getrocknet.

Eine andere Technik zur Aufbringung der Aktivkomponente besteht darin, den Träger entsprechend seiner vorher ermittelten Wasseraufnahme, die im wesentlichen seinem Porenvolumen entspricht, mit einem Metallsol zu tränken. Nach Abtropfen des Trägers wird dieser dann bei einer Temperatur, die 150°C nicht übersteigt, getrocknet. Derart hergestellte Katalysatoren weisen die Aktivkomponente überraschenderweise ebenfalls in einer sehr dünnen Schicht auf. In diesem Fall liegt die Aktivkomponente jedoch, wenn makroporöse Träger eingesetzt werden, in den von außen zugänglichen Makroporen angereichert vor, während beim Aufsprühen des Sols im wesentlichen eine Gleichverteilung der Aktivkomponente in Mikro- und Makroporen erfolgt. Ein wesentlicher Vorteil der Soltränk- und Solaufsprühtechnik besteht darin, daß die Aktivkomponente nach Aufbringen des Sols auf dem Träger im wesentlichen bereits im reduzierten Zustand vorliegt. Damit kann eine Reduktion der Aktivkomponente bei hohen Temperaturen, die im allgemeinen ein Zusammensintern der Aktivkomponente bewirkt und dadurch die katalytische Oberfläche verringert, entfallen.

Zur Durchführung des erfindungsgemäßen Verfahrens wird Vinyloxiran oder Lösungen des Vinyloxirans in einem unter den Reaktionsbedingungen inerten Lösungsmittel in Gegenwart der erfindungsgemäß zu verwendenden Katalysatoren bei Temperaturen von im allgemeinen 0 bis 200°C, vorzugsweise von 10 bis 130°C, insbesondere von 20 bis 100°C und besonders bevorzugt bei 25 bis 60°C bei einem Druck von im allgemeinen 1 bis 300 bar, vorzugsweise von 1 bis 100 bar und besonders bevorzugt von 1 bis 50 bar hydriert.

Das erfindungsgemäße Verfahren kann ohne Lösungsmittel oder vorteilhaft in Anwesenheit eines unter den Reaktionsbedingungen inerten Lösungsmittels durchgeführt werden. Derartige Lösungsmittel können beispielsweise sein: Ether wie Tetrahydrofuran, Dioxan, Methyl-tert.-butylether, Di-n-butylether, Dimethoxyethan und Diisopropylether, Alkohole wie Methanol, Ethanol, Propanol, Isopropanol, n-Butanol, Isobutanol und tert.-Butanol, C₂ - bis C₄-Glycole, Kohlenwasserstoffe wie Petrolether, Benzol, Toluol und Xylol, und N-Alkyl-Lactame wie N-Methylpyrrolidon und N-Octylpyrrolidon.

Das erfindungsgemäße Verfahren kann sowohl kontinuierlich als auch diskontinuierlich, in der Gasphase oder in der flüssigen Phase, ausgeübt werden. Bei der kontinuierlichen Betriebsweise können beispielsweise Rohrreaktoren vorteilhaft eingesetzt werden, in denen der Katalysator als Festbett angeordnet ist, über das die Reaktionsmischung in Sumpf- oder Rieselfahrweise geleistet werden kann. Bei der diskontinuierlichen Betriebsweise kann der Katalysator z.B. in Rührreaktoren in suspendierter Form oder vorteilhaft in einem Festbett angeordnet, z.B. bei Verwendung von Schlaufenreaktoren, verwendet werden.

Die Aufarbeitung der Reaktionsmischung zur Isolierung des 1,2-Butylenoxids kann auf herkömmliche Weise, z.B. durch Destillation, erfolgen.

Das als Ausgangsmaterial benötigte Vinyloxiran kann z.B. nach dem Verfahren von US-A 4,897,498 durch partielle Oxidation von 1,3-Butadien an Silberkatalysatoren hergestellt werden.

1,2-Butylenoxid findet als Kraftstoffadditiv und als Stabilisator von Chlorkohlenwasserstoffen Verwendung.

### Herstellung der Katalysatoren

### Beispiel 1: Herstellung des Katalysators A (Pd auf Al₂O₃)

Zur Darstellung eines stabilen Pd-Sols wurden 1,60 g einer 11 gew.-%igen Pd(NO₃)₂-Lösung und 5 g Polyvinylpyrrolidon in 1,17 l Wasser gelöst. Zu dieser Lösung wurden 25 ml einer 0,8 gew.-%igen N₂H₄-Lösung zugegeben, 1/2 h bei Raumtemperatur gerührt und anschließend 4 h am Rückfluß erhitzt. Nach Abkühlen wurde ein Palladiumsol mit einem Gehalt von 0,15 g Pd/l erhalten. 400 ml dieses Sols wurden mit 600 ml Wasser auf 1 l verdünnt. 100 g eines Al₂O₃-Trägers mit einer BET-Oberfläche von 290 m²/g wurden im beheizten Pelletierteller vorgelegt und mit dem zuvor hergestellten verdünnten Sol besprüht. Der Katalysator wurde 16 h bei 120°C getrocknet. Nach dieser Trockenzeit enthielt der Katalysator 0,05 Gew.-% Palladium, bezogen auf den Trägerkatalysator.

### Beispiel 2: Herstellung des Katalysators B (Pd auf Al₂O₃)

Zur Herstellung des Katalysators B wurde wie in Beispiel 1 beschrieben verfahren, mit dem Unterschied, daß nur 267 ml des unverdünnten Sols auf 1000 ml verdünnt wurden. 100 g eines Al₂O₃-Trägers mit einer BET-Oberfläche von 244 m²/g wurden im beheizten Pelletierteller vorgelegt und mit dem zuvor hergestellten verdünnten Sol besprüht. Der Katalysator wurde 16 h bei 120°C getrocknet. Nach dieser Trockenzeit enthielt der Katalysator 0,035 Gew.-% Palladium, bezogen auf den Trägerkatalysator.

### Beispiel 3: Herstellung des Katalysators C (Pd auf Al₂O₃)

Zur Darstellung eines stabilen Pd-Sols wurden 9,09 g Pd(N03)2 einer 1l gew.-%igen Lösung und 5 g Polyvinylpyrrolidon in 990 ml eines 1:1 Gemisches aus Ethanol und Wasser gelöst. Die Lösung wurde 1/2 h bei Raumtemperatur gerührt und anschließend 4 h zum Rückfluß erhitzt. Nach Abkühlen wurde ein Palladiumsol mit einem Gehalt von 1 g Pd/l erhalten. 20 ml dieses Sols wurden mit 980 ml Wasser auf 1 l verdünnt. 100 g eines Al₂O₃-Trägers mit einer BET-Oberfläche von 244 m²/g wurden im beheizten Pelletierteller vorgelegt und mit dem zuvor hergestellten verdünnten Sol besprüht. Der Katalysator wurde 16 h bei 120°C getrocknet. Nach dieser Trockenzeit enthielt der Katalysator laut Analyse 0,023 Gew.-% Palladium, bezogen auf den Trägerkatalysator.

### Beispiel 4: Herstellung des Katalysators D (Pd auf Al₂O₃)

Zur Darstellung eines stabilen Pd-Sols wurden 1,60 g einer 11 gew.-%igen Pd(NO₃)₂-Lösung und 5 g Polyvinylpyrrolidon in 1170 ml Wasser gelöst. Zu dieser Lösung wurden 25 ml einer 0,8 gew.-%igen N₂H₄-Lösung zugegeben, 1/2 h bei Raumtemperatur gerührt und anschließend 4 h zum Rückfluß erhitzt.

Nach Abkühlen wurde ein Palladiumsol mit einem Gehalt von 0,15 g Pd/l erhalten. 33 ml dieses Sols wurden mit Wasser auf 1 l verdünnt. 100 g eines Al₂O₃-Trägers mit einer BET-Oberfläche von 270 m²/g wurden im beheizten Pelletierteller vorgelegt und mit dem zuvor hergestellten verdünnten Sol besprüht. Der Katalysator wurde 16 h bei 120°C getrocknet. Nach dieser Trockenzeit enthielt der Katalysator 0,005 Gew.-% Palladium, bezogen auf den Trägerkatalysator.

### Beispiel 5: Herstellung des Katalysators E (Pd auf Steatit)

Zur Darstellung eines stabilen Pd-Sols wurden 27,27 g einer 11 gew.-%igen Pd(NO₃)₂-Lösung und 5 g Polyvinylpyrrolidon in 990 ml eines 1:1 Gemisches aus Ethanol und Wasser gelöst. Die Lösung wurde 1/2 h bei Raumtemperatur gerührt und anschließend 4 h zum Rückfluß erhitzt. Nach Abkühlen wurde ein Palladiumsol mit einem Gehalt von 3 g Pd/l erhalten. 17 ml wurden in drei Tränkstufen (2 mal 6 ml und einmal 5 ml) auf 100 g eines Steatit-Trägers (Steatit-Kugeln ohne innere Oberfläche) aufgebracht. Zwischen jedem Tränkschritt lag etwa 1 h und die Kugeln wurden während dieser Zeit mehrmals aufgeschüttelt. Der Katalysator wurde 16 h bei 120°C getrocknet. Nach dieser Trockenzeit enthielt der Katalysator 0,027 Gew.-% Palladium, bezogen auf den Trägerkatalysator.

### Beispiel 6: Herstellung des Katalysators F (Pd auf SiO₂)

1,37 g einer 11 gew.-%igen Pd(NO₃)₂-Lösung wurden mit 2,25 l Wasser gemischt und die Lösung mit 1,5 g Polyvinylpyrrolidon versetzt. Zu dieser Lösung wurden 750 ml Ethanol gegeben und die Lösung 4 h zum Rückfluß erhitzt. Nach Abkühlen wurde ein stabiles Sol erhalten. 400 ml dieses Sols wurden auf 1 l verdünnt. Das verdünnte Sol wurde im beheizten Pelletierteller auf 100 g eines SiO₂-Trägers mit einer BET-Oberfläche von 136 m²/g gesprüht. Anschließend wurde der Katalysator 16 h bei 120°C getrocknet. Der so erhaltene Katalysator hatte einen Pd-Gehalt von 0,024 %.

### Beispiel 7: Herstellung des Katalysators G (Re auf Al₂O₃)

1,127 g Rhenium(V)-chlorid wurden in 700 ml Wasser unter Zugabe von 1,5 g Polyvinylpyrrolidon gelöst. Diese Lösung wurde mit 300 ml Ethanol versetzt und 4 h am Rückfluß gerührt. Nach Abkühlen erhielt man ein stabiles Sol (0,6 g/l). 83 ml dieses Sols wurden mit Wasser auf 1 l verdünnt. 100 g eines Al₂O₃-Trägers mit einer BET-Oberfläche von 222 m²/g wurden im beheizten Pelletierteller vorgelegt und mit dem zuvor hergestellten verdünnten Sol besprüht. Der Katalysator wurde 16 h bei 120°C getrocknet. Nach dieser Trockenzeit enthielt der Katalysator 0,05 Gew.-% Rhenium, bezogen auf den Trägerkatalysator.

### Beispiel 8: Herstellung des Katalysators H (Ru auf Al₂O₃)

1,68 g Ruthenium(III)-chlorid-Hydrat wurden in 700 ml Wasser unter Zugabe von 5 g Polyvinylpyrrolidon gelöst. Diese Lösung wurde mit 300 ml Ethanol versetzt und 4 h am Rückfluß gerührt. Nach Abkühlen erhielt man ein stabiles Sol (0,6 g/l). 83 ml dieses Sols wurden mit Wasser auf 1 l verdünnt. 100 g des in Beispiel 7 beschriebenen Trägers wurden im beheizten Pelletierteller vorgelegt und mit dem zuvor hergestellten verdünnten Sol besprüht. Der Katalysator wurde 16 h bei 120°C getrocknet. Nach dieser Trockenzeit enthielt der Katalysator laut Analyse 0,05 Gew.-% Ruthenium, bezogen auf den Trägerkatalysator.

### Beispiel 9: Herstellung des Katalysators I (Rh auf Al₂O₃)

1,73 g Rhodium(III)-chlorid-Hydrat wurden in 700 ml Wasser unter Zugabe von 1 g Polyvinylpyrrolidon gelöst. Diese Lösung wurde mit 300 ml Ethanol versetzt und 4 h am Rückfluß gerührt. Nach Abkühlen erhielt man ein stabiles Sol (0,6 g/l). 83 ml dieses Sols wurden mit Wasser auf 1 l verdünnt. 100 g des in Beispiel 7 beschriebenen Trägers wurden im beheizten Pelletierteller vorgelegt und mit dem zuvor hergestellten verdünnten Sol besprüht. Der Katalysator wurde 16 h bei 120°C getrocknet. Nach dieser Trockenzeit enthielt der Katalysator 0,05 Gew.-% Rhodium, bezogen auf den Trägerkatalysator.

### Hydrierbeispiele

### Beispiel 10

In einem 50 ml-Autoklaven wurden die zu hydrierende Lösung aus 2,5 g Vinyloxiran und 22,5 g Tetrahydrofuran mit 0,5 g suspendiertem Katalysator A bei 25°C und 40 bar 8 h lang mit Wasserstoff hydriert. Nach gaschromatographischer Untersuchung des Hydrieraustrages wurden bei quantitativem Vinyloxiran-Umsatz 90 mol-% 1,2-Butylenoxid, 1,0 mol-% n-Butyraldehyd und 1,7 mol-% n-Butanol erhalten.

### Beispiele 11 bis 18

2,5 g Vinyloxiran in 22,5 g Tetrahydrofuran wurden wie in Beispiel 1 beschrieben bei 40 bar an den Katalysatoren B - I hydriert. Nachstehende Tabelle zeigt die Hydrierbedingungen, Temperatur, Druck und Reaktionszeit und die gaschromatographischen Analyseergebnisse der Hydrierausträge.

**Tabelle**

| Bsp. | Katalysator | Kat. [g] | T [°C] | Reaktionszeit [h] | VO-Umsatz [%] | Ausbeute [mol-%] | | |
|---|---|---|---|---|---|---|---|---|
| | | | | | | BO | n-BA | n-BuOH |
| 11 | B | 1 | 20 | 4 | 100 | 85 | 1 | 3 |
| 12 | C | 1 | 20 | 4 | 100 | 82 | 2 | 3 |
| 13 | D | 1 | 20 | 8 | 100 | 80 | 1 | 4 |
| 14 | E | 1 | 20 | 6 | 100 | 81 | 2 | 6 |
| 15 | F | 0,5 | 25 | 6 | 100 | 83 | 4 | 4 |
| 16 | G | 1 | 20 | 17 | 100 | 74 | 1 | 2 |
| 17 | H | 2 | 20 | 24 | 100 | 74 | 1 | 2 |
| 18 | I | 1 | 20 | 8 | 100 | 83* | 1 | 3 |
| VO =Vinyloxiran, BO = 1,2-Butylenoxid, n-BA = n-Butyraldehyd, n-BuOH = n-Butanol Bsp.= Beispiel, Kat. = Katalysator | | | | | | | | |

| | | | | | | | | |
|---|---|---|---|---|---|---|---|---|
| * Raum-Zeit-Ausbeute: 550 kg BO/kg Rh * h | | | | | | | | |

## Patentansprüche

1. Verfahren zur Herstellung von 1,2-Butylenoxid durch katalytische Hydrierung von Vinyloxiran an einem Heterogenkatalysator, dadurch gekennzeichnet, daß man einen Katalysator verwendet, der durch Aufbringen mindestens eines Elements der Gruppe Palladium, Platin, Rhodium, Rhenium oder Ruthenium in Form eines Sols auf einen inerten Träger hergestellt wird.

2. Verfahren nach Anspruch 1, dadurch gekennzeichnet, daß man einen Katalysator verwendet, der Palladium auf Aluminiumoxid enthält.

3. Verfahren nach den Ansprüchen 1 bis 2, dadurch gekennzeichnet, daß das Sol durch Reduktion eines Salzes eines Elements der Gruppe Palladium, Platin, Rhodium, Rhenium oder Ruthenium in Gegenwart von Polyvinylpyrrolidon hergestellt wird.

4. Verfahren nach Anspruch 3, dadurch gekennzeichnet, daß als Reduktionsmittel Hydrazin oder Ethanol verwendet wird.

## Claims

1. A process for preparing 1,2-butylene oxide by catalytic hydrogenation of vinyloxirane over a heterogeneous catalyst, which comprises using a catalyst prepared by applying at least one element from palladium, platinum, rhodium, rhenium or ruthenium in the form of a sol to an inert support.

2. A process as claimed in claim 1, wherein the catalyst used comprises palladium on alumina.

3. A process as claimed in claim 1 or 2, wherein the sol is prepared by reducing a salt of an element from palladium, platinum, rhodium, rhenium or ruthenium in the presence of polyvinylpyrrolidone.

4. A process as claimed in claim 3, wherein hydrazine or ethanol is used as reducing agent.

## Revendications

1. Procédé de préparation d'oxyde de 1,2-butylène par hydrogénation catalytique de vinyloxyrane sur un catalyseur d'hydrogénation, caractérisé en ce que l'on utilise un catalyseur fabriqué par dépôt sur un support inerte d'au moins un élément du groupe formé par le palladium, la platine, le rhodium, le rhénium ou le ruthénium sous forme d'un sol.

2. Procédé selon la revendication 1, caractérisé en ce que l'on utilise un catalyseur contenant du palladium sur de l'oxyde d'aluminium.

3. Procédé selon la revendication 1 ou 2, caractérisé en ce que le sol est préparé par réduction d'un sel d'un élément du groupe formé par le palladium, le platine, le rhodium, le rhénium ou le ruthénium, en présence de polyvinylpyrrolidone.

4. Procédé selon la revendication 3, caractérisé en ce que l'on utilise de l'hydrazine ou de l'éthanol en tant qu'agent de réduction.
